(19) Europäisches Patentamt
European Patent Office
Office européen des brevets

(11) Veröffentlichungsnummer: **0 036 441**
**B1**

(12) # EUROPÄISCHE PATENTSCHRIFT

(45) Veröffentlichungstag der Patentschrift: **07.09.83**

(51) Int. Cl.³: **C 07 C 120/00,** C 07 C 121/76

(21) Anmeldenummer: **80106231.6**

(22) Anmeldetag: **14.10.80**

(54) **Verfahren zur Herstellung von Carbonsäurecyaniden.**

(30) Priorität: **24.03.80 DE 3011305**

(43) Veröffentlichungstag der Anmeldung:
**30.09.81 Patentblatt 81/39**

(45) Bekanntmachung des Hinweises auf die Patenterteilung:
**07.09.83 Patentblatt 83/36**

(84) Benannte Vertragsstaaten:
**AT BE CH DE FR GB IT LI LU NL SE**

(56) Entgegenhaltungen:
**DE-A-2 624 891**
**DE-A-2 708 182**

**Die Akte enthält technische Angaben, die nach dem Eingang der Anmeldung eingereicht wurden und die nicht in dieser Patentschrift enthalten sind.**

(73) Patentinhaber: **Degussa Aktiengesellschaft, Weissfrauenstrasse 9, D-6000 Frankfurt am Main 1 (DE)**

(72) Erfinder: **Kleemann, Axel, Dr., Greifenhagenstrasse 9, D-6450 Hanau 9 (DE)**
Erfinder: **Lehmann, Bernd, Dr., Flurstrasse 5, D-6450 Freigericht (DE)**
Erfinder: **Klenk, Herbert, Dr., Grünaustrasse 19, D-6450 Hanau 9 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 036 441 B1

BUNDESDRUCKEREI BERLIN

Verfahren zur Herstellung von Carbonsäurecyaniden

Die Erfindung betrifft ein Verfahren zur Herstellung von Carbonsäurecyaniden durch Umsetzung von Carbonsäurehalogeniden mit Alkalicyaniden oder Cyanwasserstoff in Gegenwart von Kupfer(I)-salzen und Carbonsäurenitrilen.

Es ist bekannt, Carbonsäurecyanide aus Carbonsäurehalogeniden zu erzeugen, indem diese in Gegenwart katalytischer Mengen von Kupfer(I)-cyanid, Kupfer(II)-cyanid, Zinkcyanid oder deren Komplexverbindungen mit Alkalicyaniden oder Cyanwasserstoff bei Temperaturen von 100 bis 300°C umgesetzt werden (DE-AS 26 14 242). Nachteilig ist bei diesem Verfahren, daß zur Erzielung brauchbarer Umsätze verhältnismäßig hohe Temperaturen, vornehmlich über 200°C, und beziehungsweise oder lange Umsetzungszeiten benötigt werden, unter diesen Bedingungen aber Nebenreaktionen, insbesondere die Bildung von Dimeren, begünstigt werden, so daß die Ausbeuten mäßig sind. Bei bestimmten Carbonsäurecyaniden, beispielsweise bei den niederen aliphatischen Carbonsäurecyaniden, betragen die Ausbeuten nur wenige Prozent.

Es ist auch bekannt, die Umsetzung der Carbonsäurehalogenide mit den Alkalicyaniden in Gegenwart von Kupfer(I)-salzen und Carbonsäurenitrilen, insbesondere Acetonitril, durchzuführen und hierbei bei Temperaturen von 50 bis 180°C zu arbeiten (DE-OS 27 08 182). Bei diesem Verfahren genügen zwar verhältnismäßig niedrige Temperaturen, die Umsetzungszeiten sind jedoch unerwünscht lang und die Ausbeuten an den Carbonsäurecyaniden nicht befriedigend.

Es ist nun ein Verfahren zur Herstellung von Carbonsäurecyaniden durch Umsetzung von Carbonsäurehalogeniden mit Alkalicyaniden oder Cyanwasserstoff in Gegenwart von Kupfer(I)-salzen und Carbonsäurenitrilen gefunden worden, das dadurch gekennzeichnet ist, daß Carbonsäurenitrile der Formel

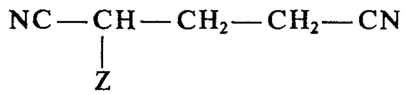 (I)

in der Z ein Wasserstoffatom oder eine Methylgruppe bedeutet, verwendet werden. Obwohl bei diesem Verfahren bei gleichen Temperaturen wie bei dem Verfahren nach der DE-OS 27 08 182 gearbeitet wird, genügen sehr viel kürzere Umsetzungszeiten zur Erreichung hoher Umsätze. Es werden erheblich bessere Ausbeuten an den Carbonsäurecyaniden erzielt. Auch die niederen aliphatischen Carbonsäurecyanide, wie das Acetylcyanid, sind nach diesem Verfahren mit ausgezeichneten Ausbeuten herstellbar.

Mit Vorteil dient das erfindungsgemäße Verfahren für die Herstellung von Carbonsäurecyaniden der Formel

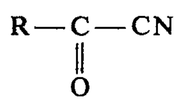 (II)

in der R einen unverzweigten oder verzweigten Alkylrest mit vorzugsweise 1 bis 7 und insbesondere 1 bis 4 Kohlenstoffatomen, einen Cycloalkylrest mit vorzugsweise 3 bis 6 und insbesondere 3 Kohlenstoffatomen im Ring, einen einfach oder mehrfach durch Methylgruppen substituierten Cycloalkylrest mit vorzugsweise 3 bis 6 Kohlenstoffatomen im Ring, einen Phenylrest, einen Furylrest oder einen Thienylrest bedeutet, durch Umsetzung von Carbonsäurehalogeniden der Formel

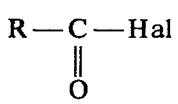 (III)

in der R die zuvor angegebenen Bedeutungen hat und Hal für ein Bromatom oder vorzugsweise für ein Chloratom steht.

Derartige Carbonsäurehalogenide sind beispielsweise Acetylbromid, Propionsäurebromid, Buttersäurebromid, Isobuttersäurebromid, Valeriansäurechlorid, Valeriansäurebromid, Isovaleriansäurebromid, 2-Methylbuttersäurebromid, 2,2-Dimethylbuttersäurebromid, Pivalinsäurebromid, Heptansäurechlorid, Cyclohexancarbonsäurechlorid, 1-Methylcyclohexancarbonsäurechlorid, Cyclopentancarbonsäurechlorid, Benzoylchlorid, Furan-2-carbonsäurechlorid und Thiophen-2-carbonsäurechlorid, vorzugsweise Buttersäurechlorid, Isovaleriansäurechlorid, 2-Methylbuttersäurechlorid, 2,2-Dimethylbuttersäurechlorid, Cyclopropancarbonsäurechlorid und 1-Methylcyclopropancarbonsäurechlorid, insbesondere Acetylchlorid, Propionsäurechlorid, Isobuttersäurechlorid und Pivalinsäurechlorid.

Von den Alkalicyaniden werden Natriumcyanid und Kaliumcyanid bevorzugt. Es ist vorteilhaft, das Alkalicyanid in möglichst feinteiliger Form, vorzugsweise als Suspension in dem Carbonsäurenitril, einzusetzen.

Es kommen sowohl einfache als auch komplexe Kupfer(I)-salze in Frage, insbesondere beispielsweise Kupfer(I)-cyanid, Kupfer(I)-chlorid, Kupfer(I)-bromid und Kaliumtetracyanocuprat (I). Es können auch solche anderen Kupfer(I)-verbindungen verwendet werden, die unter den Umsetzungsbedingungen in Kupfer(I)-salze übergehen, wie beispielsweise Kupfer(I)-oxid. In manchen Fällen, insbesondere wenn die Umsetzung mit Cyanwasserstoff durchgeführt wird, ist es vorteilhaft, Kupfermetall, zweckmäßigerweise in Pulverform, zuzusetzen. In diesen Fällen können Kupfer(II)-verbindungen anstelle von Kupfer(I)-verbindungen angewendet werden.

Die Umsetzung erfolgt erfindungsgemäß in Gegenwart von Carbonsäurenitrilen gemäß der Formel I. Diese sind das Glutarsäuredinitril und das 2-Methylglutarsäuredinitril. Vorzugsweise wird das 2-Methylglutarsäuredinitril verwendet.

In einigen Fällen kann es vorteilhaft sein, zur Verdünnung inerte organische Lösungsmittel zuzusetzen. Als Lösungsmittel kommen beispielsweise Äther, wie Dioxan oder Äthylenglykoläther, oder Ester, wie Butylacetat, in Frage. Besonders geeignet sind aromatische Kohlenwasserstoffe, wie Benzol, Toluol, Xylole oder Tetralin, oder aliphatische Kohlenwasserstoffe, wie Ligroin mit einem Siedebereich etwa von 90 bis 140°C oder Cyclohexan, oder halogenierte, vorzugsweise chlorierte, aromatische oder aliphatische Kohlenwasserstoffe, wie Trichloräthylen und insbesondere Chlorbenzol, Dichlorbenzole oder Tetrachloräthan. Auch Gemische derartiger Lösungsmittel können verwendet werden.

In welchen Mengenverhältnissen die Substanzen, nämlich die Carbonsäurehalogenide, die Alkalicyanide oder der Cyanwasserstoff, die Carbonsäurenitrile und die Kupfer(I)-salze, angewendet werden und welche Umsetzungsbedingungen, wie Temperatur und Druck, gewählt werden, richtet sich nach der Art der Substanzen sowie gegebenenfalls nach der Art und Menge des Lösungsmittels.

Es ist im allgemeinen zweckmäßig, wenigstens etwa stöchiometrische Mengen Cyanid — Alkalicyanid oder Cyanwasserstoff — zu nehmen. Vorteilhaft ist die Anwendung von 1,05 bis 3,0 Äquivalenten Cyanid, insbesondere von 1,05 bis 1,5 Äquivalenten Cyanid, je Mol Carbonsäurehalogenid. Außerdem ist es im allgemeinen zweckmäßig, je Mol Carbonsäurehalogenid 0,05 bis 1,0 Äquivalente der Kupfer(I)-salze zu nehmen. Zu bevorzugen sind je Mol Carbonsäurehalogenid 0,05 bis 0,5 Äquivalente der Kupfer(I)-salze. Wird als Kupfer(I)-salz das Cyanid verwendet, so kann ganz oder teilweise eine äquivalente Menge Alkalicyanid oder Cyanwasserstoff eingespart werden. Es ist aber im allgemeinen vorteilhaft, nicht mehr als 0,5 Äquivalente Cyanid in Form von Kupfer(I)-cyanid einzubringen. Wenngleich das Kupfermetall in weitgehend beliebigen Mengen angewendet werden kann, ist es im allgemeinen zweckmäßig, nicht wesentlich mehr als eine den vorgesehenen Kupfer(I)-verbindungen äquivalente Menge Kupfermetall einzusetzen.

Von den Carbonsäurenitrilen werden zweckmäßigerweise wenigstens 0,05 Mol je Mol Carbonsäurehalogenid eingesetzt. Wenngleich das Nitril in vielfach molarem Überschuß angewendet werden kann, ist es vorteilhaft, nicht mehr als 2 Mol Nitril je Mol Carbonsäurehalogenid zu nehmen. Zu bevorzugen sind je Mol Carbonsäurehalogenid 0,1 bis 1,5 Mol Nitril, insbesondere 0,1 bis 1,0 Mol Nitril.

Die Umsetzung wird im allgemeinen bei Temperaturen von 50 bis 180°C, vorzugsweise bei Temperaturen von 70 bis 130°C, ausgeführt. Wenngleich der Druck weitgehend beliebig gewählt werden kann, ist es vorteilhaft, vom Normaldruck nicht wesentlich abzuweichen. In manchen Fällen kann es wegen des Vorliegens flüchtiger Substanzen zweckmäßig sein, einen der Temperatur entsprechenden höheren Druck zu wählen. Es kann außerdem vorteilhaft sein, unter Ausschluß von Sauerstoff zu arbeiten.

Im Falle der Umsetzung mit Alkalicyanid ist eine bevorzugte Verfahrensweise, die insbesondere bei der Durchführung des Verfahrens in technischem Maßstab mit Vorteil angewendet wird, das Carbonsäurehalogenid vorzulegen, dieses zunächst auf die Umsetzungstemperatur zu erwärmen und dann bei dieser Temperatur das Alkalicyanid einzutragen. Hierbei ist es außerdem günstig, das Kupfer(I)-salz mit dem Carbonsäurehalogenid vorzulegen und das Alkalicyanid als Feststoff, suspendiert in dem Carbonsäurenitril, zuzuführen. Im Falle der Umsetzung mit Cyanwasserstoff ist eine bevorzugte Verfahrensweise, eine Mischung des Carbonsäurehalogenids mit dem Carbonsäurenitril, der Kupferverbindung und gegebenenfalls dem Kupfermetall vorzulegen, diese Mischung auf die Umsetzungstemperatur zu erwärmen und in die warme Mischung den Cyanwasserstoff einzubringen.

## Beispiel 1

Eine Mischung aus 628 g (8,0 Mol) Acetylchlorid, 510 g (10,4 Mol) Natriumcyanid, 72 g (0,8 Mol) Kupfer(I)-cyanid und 800 g (7,4 Mol) 2-Methylglutarsäuredinitril wurde 4 Stunden lang unter Rückfluß auf Siedetemperatur gehalten. Danach wurde abgekühlt und unter Absaugen filtriert. Der Rückstand wurde mit 500 ml 2-Methylglutarsäuredinitril gewaschen. Das Filtrat wurde destilliert. Das Acetylcyanid, das hierbei gewonnen wurde, war, wie durch Gaschromatographie und argentometrische Cyanid-Titration festgestellt wurde, 99%ig. Der Siedepunkt des Acetylcyanids war 93°C. Die Ausbeute betrug 375 g, entsprechend 67%, bezogen auf das eingesetzte Acetylchlorid.

## Beispiel 2

Eine Mischung aus 92,5 g (1,0 Mol) Propionsäurechlorid, 68,6 g (1,4 Mol) Natriumcyanid, 9,0 g (0,1 Mol) Kupfer(I)-cyanid und 76 g (0,7 Mol) 2-Methylglutarsäuredinitril wurde 1 Stunde lang unter Rückfluß auf Siedetemperatur gehalten. Danach wurde abgekühlt und unter Absaugen filtriert. Der Rückstand wurde mit 76 g 2-Methylglutarsäuredinitril gewaschen. Das Filtrat wurde unter vermindertem Druck destilliert. Es wurden hierbei 66 g eines 97%igen Propionsäurecyanids gewonnen. Dies entsprach einer Ausbeute an Propionsäurecyanid von 77%, bezogen auf eingesetztes Propionsäurechlorid.

## Beispiel 3

Eine Mischung aus 106,5 g (1,0 Mol) Buttersäurechlorid, 68,6 g (1,4 Mol) Natriumcyanid, 9,0 g (0,1 Mol) Kupfer(I)-cyanid, 14 g (0,13 Mol) 2-Methylglutarsäuredinitril und 29 g Tetralin wurde 2 Stunden lang unter Rückfluß auf Siedetemperatur gehalten. Das Umsetzungsgemisch wurde danach unter vermindertem Druck destilliert. Hierbei wurden 79 g eines 97%igen Buttersäurecyanids gewonnen. Dies entsprach einer Ausbeute an Buttersäurecyanid von 79%, bezogen auf das eingesetzte Buttersäurechlorid.

## Beispiel 4

241,2 g (2,0 Mol) Pivalinsäurechlorid wurden mit 17,9 g (0,2 Mol) Kupfer(I)-cyanid auf Siedetemperatur erhitzt. Im Verlauf einer Stunde wurde bei dieser Temperatur unter Rühren eine Suspension von 105,9 g (2,2 Mol) Natriumcyanid in 142,5 (1,3 Mol) 2-Methylglutarsäuredinitril zugesetzt. Die Mischung wurde 30 Minuten lang auf Temperaturen bis 130°C gehalten. Danach wurde abgekühlt und filtriert. Der Rückstand wurde mit 120 ml 2-Methylglutarsäuredinitril gewaschen. Bei der Destillation des Filtrats ergaben sich 209 g 98%iges Pivalinsäurecyanid. Dies entsprach einer Ausbeute an Pivalinsäurecyanid von 94%, bezogen auf das eingesetzte Pivalinsäurechlorid.

## Beispiel 5

Es wurde wie nach Beispiel 4 verfahren, jedoch wurden statt Pivalinsäurechlorid 213,1 g (2,0 Mol) Isobuttersäurechlorid eingesetzt. Bei der Destillation ergaben sich 155 g reines Isobuttersäurecyanid, entsprechend einer Ausbeute von 80%, bezogen auf das eingesetzte Isobuttersäurechlorid.

## Beispiel 6

Es wurde wie nach Beispiel 2 verfahren, jedoch wurden statt Propionsäurechlorid 120,5 g (1,0 Mol) 2-Methylbuttersäurechlorid eingesetzt und die Mischung wurde 1,5 Stunden auf Temperaturen bis 130°C gehalten. Bei der Destillation ergaben sich 98 g reines 2-Methylbuttersäurecyanid, entsprechend einer Ausbeute von 88%, bezogen auf das eingesetzte 2-Methylbuttersäurechlorid.

## Beispiel 7

Es wurde wie nach Beispiel 2 verfahren, jedoch wurden 120,5 g (1,0 Mol) 3-Methylbuttersäurechlorid eingesetzt und die Mischung wurde 1,5 Stunden auf Temperaturen bis 130°C gehalten. Bei der Destillation ergaben sich 100 g eines 96%igen 3-Methylbuttersäurecyanids. Dies entsprach einer Ausbeute an 3-Methylbuttersäurecyanid von 86%, bezogen auf das eingesetzte 3-Methylbuttersäurechlorid.

## Beispiel 8

Es wurde wie nach Beispiel 2 verfahren, jedoch wurden statt Methylglutarsäuredinitril 76 g (0,8 Mol) Glutarsäuredinitril eingesetzt und die Mischung wurde 1,5 Stunden lang unter Rückfluß auf Siedetemperatur gehalten. Bei der Destillation ergaben sich 64 g eines 97%igen Propionsäurecyanids. Dies entsprach einer Ausbeute an Propionsäurecyanid von 75%, bezogen auf das eingesetzte Propionsäurechlorid.

## Beispiel 9

Eine Mischung aus 104,5 g (1,0 Mol) Cyclopropancarbonsäurechlorid, 68,6 g (1,4 Mol) Natriumcyanid, 9,0 g (0,1 Mol) Kupfer(I)-cyanid und 67 g 2-Methylglutarsäuredinitril wurde 1 Stunde lang unter Rühren auf Temperaturen von 120 bis 130°C gehalten. Danach wurde abgekühlt und filtriert. Der Rückstand wurde mit 67 g 2-Methylglutarsäuredinitril gewaschen. Das Filtrat wurde unter vermindertem Druck destilliert. Es wurden hierbei 78 g 99%iges Cyclopropancarbonsäurecyanid gewonnen, entsprechend einer Ausbeute von 81%, bezogen auf das eingesetzte Cyclopropancarbonsäurechlorid. Der Siedepunkt des Cyclopropancarbonsäurecyanids war 78°C bei 88 mbar.

## Beispiel 10

Es wurde wie nach Beispiel 9 verfahren, jedoch wurden statt Cyclopropancarbonsäurechlorid 140,5 g (1,0 Mol) Benzoylchlorid eingesetzt. Bei der Destillation ergaben sich 122 g 97%iges Benzoylcyanid, entsprechend einer Ausbeute von 91%, bezogen auf das eingesetzte Benzoylchlorid. Der Siedepunkt des Benzoylcyanids war 93°C bei 16 mbar.

## Beispiel 11

Eine Mischung aus 120,6 g (1,0 Mol) Pivalinsäurechlorid, 9,0 g (0,1 Mol) Kupfer(I)-cyanid, 6,4 g (0,1 g-Atom) gepulvertem Kupfer und 150 ml 2-Methylglutarsäuredinitril wurde im Verlauf von 6 Stunden mit 29,7 g (1,1 Mol) Cyanwasserstoff versetzt. Die Mischung wurde währenddessen unter Stickstoff auf Temperaturen von 90 bis 110°C gehalten. Es entwich hierbei Chlorwasserstoff und Cyanwasserstoff; der Cyanwasserstoff wurde kondensiert und zurückgeführt. Aus dem Umsetzungsgemisch wurden durch fraktionierte Destillation unter vermindertem Druck 10 g Pivalinsäurechlorid und 96 g 98%iges Pivalinsäurecyanid gewonnen. Die Ausbeute an Pivalinsäurecyanid betrug 85%, bezogen auf das eingesetzte Pivalinsäurechlorid. Für weitere Ansätze wurde jeweils der bei der Destillation des vorigen Umsetzungsgemischs verbliebene Rückstand verwendet. Diesem wurden zunächst 120,6 g (1,0 Mol) Pivalinsäurechlorid und dann im Verlauf von 6 Stunden 29,7 g (1,1 Mol) Cyanwasserstoff zugesetzt. Im übrigen wurde wie zuvor beschrieben verfahren. Die Ausbeuten an Pivalinsäurecyanid betrugen 86 bis 88%, bezogen auf das eingesetzte Pivalinsäurechlorid.

## Beispiel 12

Eine Mischung aus 120,6 g (1,0 Mol) Pivalinsäurechlorid, 9,0 g (0,1 Mol) Kupfer(I)-cyanid, 6,4 g (0,1 g-Atom) gepulvertem Kupfer, 150 ml 2-Methylglutarsäuredinitril und 29,7 g (1,1 Mol) Cyanwasserstoff wurde unter Stickstoff in einem Druckgefäß auf 115°C erwärmt und 3,5 Stunden lang auf dieser Temperatur gehalten. Der Druck in dem Gefäß war währenddessen auf 3 bar eingeregelt, und hierbei wurde mittels Kühlung so verfahren, daß Chlorwasserstoff, nicht aber Cyanwasserstoff, entwich. Bei der Destillation des Umsetzungsgemischs wurden 9 g Pivalinsäurechlorid und 98 g 98%iges Pivalinsäurecyanid gewonnen. Die Ausbeute an Pivalinsäurecyanid, bezogen auf das eingesetzte Pivalinsäurechlorid, betrug 87%.

## Beispiel 13

Es wurde wie nach Beispiel 12 verfahren, jedoch wurde der Druck auf 6 bar gehalten. Gewonnen wurden 7 g Pivalinsäurechlorid und 99 g 98%iges Pivalinsäurecyanid, entsprechend 89% Ausbeute, bezogen auf das eingesetzte Pivalinsäurechlorid.

## Beispiel 14

In 110 g 2-Methylglutarsäuredinitril wurden gleichzeitig 29,7 g (1,1 Mol) Cyanwasserstoff und 198 g einer 30%igen Lösung von Natriummethylat in Methanol (entsprechend 1,1 Mol Natriummethylat) eindosiert. Aus der Mischung wurde das Methanol unter vermindertem Druck abgetrieben. Die so erzeugte Natriumcyanid-Suspension wurde innerhalb von 5 Minuten in eine Mischung aus 120,6 g (1,0 Mol) Pivalinsäurechlorid und 9,0 g (0,1 Mol) Kupfer(I)-cyanid eingetragen. Die Temperatur der Mischung wurde hierbei und weitere 10 Minuten lang auf 125 bis 130°C gehalten. Gewonnen wurden 107 g 98%iges Pivalinsäurecyanid, entsprechend einer Ausbeute von 95%, bezogen auf das eingesetzte Pivalinsäurechlorid.

**Patentansprüche**

1. Verfahren zur Herstellung von Carbonsäurecyaniden der Formel

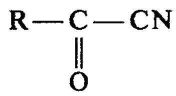

durch Umsetzung von Carbonsäurehalogeniden der Formel

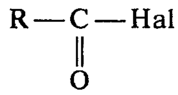

wobei R einen unverzweigten oder verzweigten Alkylrest, einen Cycloalkylrest, einen einfach oder mehrfach durch Methylgruppen substituierten Cycloalkylrest, einen Phenylrest, einen Furylrest oder einen Thienylrest bedeutet, mit Alkalicyaniden oder Cyanwasserstoff in Gegenwart von Kupfer(I)-salzen und Carbonsäurenitrilen, dadurch gekennzeichnet, daß Carbonsäurenitrile der Formel

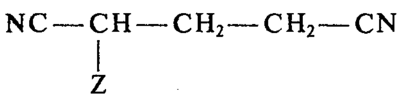

in der Z ein Wasserstoffatom oder eine Methylgruppe bedeutet, verwendet werden.

2. Verfahren nach Anspruch 1, dadurch gekennzeichnet, daß je Mol Carbonsäurehalogenid 0,1 bis 1,5 Mol des Carbonsäurenitrils eingesetzt werden.

**Claims**

1. A process for the production of carboxylic acid cyanides corresponding to the formula

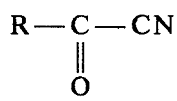

by the reaction of carboxylic acid halides corresponding to the formula

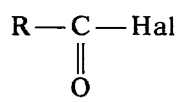

wherein R represents an unbranched or branched alkyl radical, a cycloalkyl radical, a cycloalkyl radical mono- or poly- substituted by methyl groups, a phenyl radical, a furyl radical or a thienyl radical, with alkali metal cyanides or hydrogen cyanide in the presence of copper-(I)-salts and carboxylic acid nitriles, characterised in that carboxylic acid nitriles are used which correspond to the formula

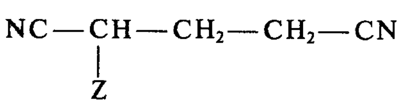

wherein Z represents a hydrogen atom or a methyl group.

2. A process according to claim 1, characterised in that from 0.1 to 1.5 mols of the carboxylic acid nitrile are used per mol of carboxylic acid halide.

**Revendications**

1. Procédé de préparation de cyanures d'acides carboxyliques de formule:

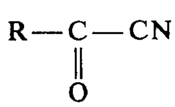

par réaction d'halogénures d'acides carboxyliques de formule:

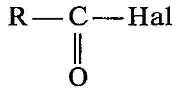

dans laquelle R signifie un reste alcoyle à chaîne droite ou ramifiée, un reste cycloalcoyle, un reste cycloalcoyle substitué une ou plusieurs fois par des groupes méthyle, un reste furyle ou un reste thiényle, avec des cyanures alcalins ou de l'acide cyanhydrique, en présence de sels de cuivre (I) et de nitriles d'acide carboxyliques, procédé caractérisé en ce que l'on utilise des nitriles d'acides carboxyliques de formule:

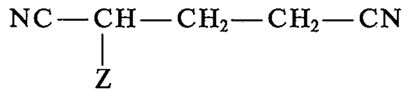

dans laquelle Z signifie un atome d'hydrogène ou un groupe méthyle.

2. Procédé suivant la revendication 1, caractérisé en ce que l'on met en oeuvre de 0,1 à 1,5 moles de nitrile d'acide carboxylique pour chaque mole d'halogénure d'acide carboxylique.